# EUROPEAN PATENT SPECIFICATION

(11) **EP 3 058 362 B1**
(45) Date of publication and mention of the grant of the patent: **02.01.2019**
(21) Application number: 14854678.1
(22) Date of filing: 15.10.2014
(51) Int. Cl.: G01N 33/15, G01N 33/53, G01N 33/68, C07K 16/06

(54) **IN-VITRO METHOD FOR DETERMINING FATE OF POLYPEPTIDE VARIANT**
IN-VITRO-VERFAHREN ZUR BESTIMMUNG DES SCHICKSALS EINER POLYPEPTIDVARIANTE
MÉTHODE IN VITRO POUR LA DÉTERMINATION DU DEVENIR D'UN VARIANT POLYPEPTIDIQUE

(30) Priority: 18.10.2013 IN 4702CH2013
(43) Date of publication of application: 24.08.2016
(73) Proprietor: Dr. Reddy's Laboratories Limited, Telangana, Hyderabad 500 034 (IN)
(72) Inventor: MEDISETTY, Rajesh, Hyderabad 500035 (IN); RAVI KUMAR, Lella, Andhra Pradesh ChiralaMandal 523165 (IN); KHAMBHAMPATY, Sridevi, Hyderabad 500090 (IN)
(74) Representative: Bates, Philip Ian
(86) International application number: PCT/IB2014/065329
(87) International publication number: WO 2015/056187

(56) References cited:
- WO-A1-2011/159878
- WO-A2-02/053761
- WO-A2-2012/007324
- US-A1- 2012 329 997
- LIU W ET AL: "Tracking the in vivo fate of recombinant polypeptides by isotopic labeling", JOURNAL OF CONTROLLED RELEASE, ELSEVIER, AMSTERDAM, NL, vol. 114, no. 2, 28 August 2006 (2006-08-28), pages 184-192, XP024957589, ISSN: 0168-3659, DOI: 10.1016/J.JCONREL.2006.06.001 [retrieved on 2006-08-28]
- DAI ET AL: "In vitro methods to assess drug precipitation", INTERNATIONAL JOURNAL OF PHARMACEUTICS, ELSEVIER, AMSTERDAM, NL, vol. 393, no. 1-2, 30 June 2010 (2010-06-30), pages 1-16, XP027065365, ISSN: 0378-5173, DOI: 10.1016/J.IJPHARM.2010.03.040 [retrieved on 2010-05-29]
- EFSTATHIOS K. ET AL.: 'Ratio of involved/uninvolved immunoglobulin quantification by Hevylite? assay: clinical and prognostic impact in multiple myeloma' EXPERIMENTAL HEMATOLOGY & ONCOLOGY vol. 1, no. 1, 23 April 2012, pages 9 - 15, XP021125940

## Description

### BACKGROUND

The invention describes a method for characterization of the fate of protein variants when administered into a subject, wherein the method is performed in-vitro, but is a predictor of an in-vivo outcome.

Recombinant immunoglobulins have found immense applications in the field of protein based therapeutics. However, production of recombinant immunoglobulins, at an industrial scale, results in formation of numerous immunoglobulin isoforms and variants. These variants include immunoglobulin molecules bearing varied charge, conformation, or glycosylation patterns.

Numerous intrinsic and extrinsic factors are responsible for generation of these variants, including post-translational modification, cell culture conditions, storage buffer conditions etc.

The significance of analyzing these immunoglobulin variants is the premise that these variants may behave differently in-vivo, and therefore influence pharmacokinetic and pharmacodynamic properties of a therapeutic immunoglobulin preparation. For instance, immunoglobulin preparations rich in high mannose glyco-variants are known to clear faster from the serum when compared to other glyco-variants *(Goetze et al.,* 25 *2011).* Similarly, afucosylated glyco-variants of immunoglobulins have been associated with enhanced ADCC activity and enhanced affinity to Fc-receptor *(Junttilla et al., 2010).* Even though no overall biological effects have been ascribed to charge variants, it is nevertheless necessary to understand and assess any potential effects of these variants.

Though the variants of a protein's in-vivo effect are ideally tested in an in vivo setting, due to the inherent constrains associated with an in-vivo assay, it is often necessary to design an in-vitro assays that "mimic" in-vivo outcomes.

The principle object of the present invention is to describe an in-vitro method to determine the fate of immunoglobulin variants in a human subject.

### SUMMARY

The present invention discloses a method for determining the fate of polypeptide variants in a physiological matrix, wherein the method is performed in an in-vitro setting.

### BRIEF DESCRIPTION OF DRAWINGS

Figure1: Depicts proportions of various charge variants present in two antibody preparations (Ab1 and Ab2) prior to incubation with immunoglobulin depleted serum.
Figure 2: Depicts proportions of various charge variants present in two distinct antibody preparations (Ab1 and Ab2) of the same therapeutic agent after incubation with immunoglobulin depleted serum.
Figure 3: Depicts the fate of acidic, basic and main variant of an
   antibody preparation in time.

### DETAILED DESCRIPTION

Various embodiments of the disclosed invention provide an in vitro method of determining the fate of immunoglobulin charge variants of a polypeptide preparation.

As described, immunoglobulin preparations produced by recombinant means typically have numerous isoforms and variants of the protein, including glyco, charge, and conformational variants. The preferred approach for assessing the fate of these variants is to analyze these variants in an in-vivo setting. However, due to inherent constrains associated with in-vivo testing methods, there is a need to develop in-vitro analytical methods that most proximally mimic a physiological matrix.

Immunoglobulins, in particular IgGs, are a major fraction of the serum protein (upto 15mg/ml of serum). The charge variants of an immunoglobulin arise due to sialylation, deamidation, cleavage of C-terminal lysine (Lyubarskay et al 2006).

The primary variants include acidic variants (negatively charged) or basic variants (positively charged). For instance, sialylation of the protein renders a net negative charge to the protein. Similarly presence of a C-terminal lysine on the heavy chain of an immunoglobulin can confer a net positive charge to the protein. The extent of the net positive charge can vary based on the number of C-terminal lysine residues present. Three main variants have been identified: namely, "K1", in which one of the heavy chain C-terminal lysine residues is cleaved, "K2", in which both heavy chain C-terminal lysine residues are preserved, and "K0", in which both heavy chain C-terminal lysine residues are cleaved.

While, the presence of these charge variants in a therapeutic protein preparation is well recognized, the fate of these variants, when in contact with a physiological matrix, needs to be better understood.

Since, immunoglobulins constitute a significant proportion of the serum; classically, serum immunoglobulin depletion has been employed to study minor protein fractions present in the serum. Further, immunoglobulin depleted serum has been utilized for culturing sensitive hybridomas, so that non-specific immunoglobulin mediated effects could be obviated. However, serum immunoglobulin depletion has not been employed to study fate of polypeptide variants, in particular charge variants, present in a therapeutic polypeptide preparation, neither has the method been used to establish bioequivalence between two or more therapeutic preparations of the same therapeutic agent.

The claimed invention discloses an in vitro method for
determining the fate of immunoglobulin charge variants of a polypeptide
preparation, comprising;
a. Isolating serum or plasma from blood.
b. Depleting immunoglobulin from the said serum or plasma,
c. Spiking the immunoglobulin depleted serum or plasma with the polypeptide preparation
d. Incubating the spiked serum or plasma of step c for a first period of time
e. Isolating immunoglobulin and immunoglobulin charge variants thereof from the spiked serum or plasma of step d, and
f. Quantifying the immunoglobulin and immunoglobulin charge variants isolated in step e.

In a further embodiment, the polypeptide preparation is a therapeutic polypeptide preparation. In a further embodiment, the therapeutic polypeptide preparation is an immunoglobulin preparation.

A number of statistical methods are known in the art to establish comparability between two or more test preparations, such as standard deviation and Student's T-test etc.

A number of techniques and commercial kits are available for serum immunoglobulin depletion as well as for isolation of Ig G from a immunoglobulin G spiked serum or plasma, such as Qproteome Albumin/IgG Depletion Kit, ProteoPrep® IgG depletion kit etc. In absence of commercially available kits, immunoglobulins could be depleted from the serum or plasma by incubating the serum with a protein A or protein G affinity matrix for a suitable amount of time and utilizing the flow-through as the immunoglobulin depleted serum. To ensure effective depletion of immunoglobulin from the serum or plasma, the serum or plasma could be repeatedly incubated with an immunoglobulin affinity matrix.

The amount of polypeptide preparation for spiking may be equivalent to or greater than the serum polypeptide concentration in the subject in
whom the polypeptide preparation is administered. The serum polypeptide concentration of a preparation can vary and is dependent on the amount of the preparation administered into a subject. For instance, if administration of 200mg/m² of a polypeptide preparation leads to a serum polypeptide concentration of 100µg/ml, then the immunoglobulin depleted serum is to be spiked with at least about 100µg/ml of the preparation or more.

The immunoglobulin and immunoglobulin charge variants thereof may be isolated from the immunoglobulin depleted serum by means of a matrix that bears substantial affinity for the said immunoglobulin and immunoglobulin charge variants thereof.

The various isoforms obtained may be quantified
physiochemically or biologically. Physiochemical quantification may encompass, without any limitation, methods such as analytical HPLC, isoelectric focusing etc. that disclose physiochemical parameters of a protein. Similarly, biological quantification may include, without any limitation, methods such as antibody dependent cytotoxicity, complement mediated cytotoxicity, in-vitro or in-vivo proliferation assays etc. that disclose biological implications of the polypeptide preparation.

The said polypeptide preparation may be a therapeutic polypeptide preparation, wherein the preparation contains one or more variants of the polypeptide.

The immunoglobulin preparation may be a therapeutic
preparation, wherein the preparation contains one or more variants of the said immunoglobulin. The immunoglobulin preparation may be any isotype of IgG.

### Example 1

Fate of basic charge variants of two therapeutic antibody preparations of the same therapeutic agent, differing in composition of their charge variants from each other, were compared by incubating these preparations in immunoglobulin depleted serum. The individual antibody preparations were incubated with immunoglobulin depleted serum, followed by isolation of the antibody preparation from the initially spiked serum and quantification of the charge variants of the antibody in both samples.

### Isolating and depleting IgG from serum or plasma

Human serum was freshly prepared from blood, and about 2.3 ml of the serum was incubated with about 1 ml of Protein A/G beads (Cat# 89958, Thermo scientific) for about one and half hours at room temperature. At the end of the incubation, the beads were allowed to settle and the harvested supernatant was and again incubated with about 1 ml of Protein A/G beads 9 Cat# 89958, Thermo Scientific) for another one and a half hour. At the end of the second incubation the serum supernatant was again harvested. This was deemed to be the IgG depleted serum. Similar method could be followed if IgG were to be depleted from the plasma.

### Spiking the IgG depleted serum or plasma with the polypeptide preparation and incubation

The IgG depleted serum was split into three aliquots, and out of these three, in two separate IgG depleted serum aliquots, about 1mg of a first test therapeutic antibody preparation (Ab1), and second test therapeutic antibody preparation (Ab2), were added respectively. The amount 1mg was chosen based on the expected serum concentration of the antibody, if it were administered into a human subject.

Once spiked, the samples were incubated at physiological temperature (about 37°C) for different periods of time. No antibody was added to the control (C) vial.

### Isolation of the test polypeptide

At the end of the incubation all samples Ab1, Ab2 and C were, again incubated with about 0.2ml of Protein A/G (Cat# 89954, Thermo scientific) for 1.5 hrs at RT. At the end of the incubation Protein A/G matrix was washed at least four times with phosphate buffered saline. The antibody bound to the protein A/G matrix was eluted at low pH by using 20mM Phosphoric acid (pH 1.9-2.3) followed by instant neutralization using 0.2M disodium hydrogen phosphate (pH 8.9-9.5).

Complete depletion of serum immunoglobulin could be confirmed at this stage, since from the control sample (C) no antibody could be eluted upon incubation with protein A/G matrix, while antibody could be isolated from the spiked serum sample (Table 1) - Represents the amount of antibody obtained either from the control samples or one of the spiked IgG depleted serum samples, following the isolation step of the test antibody preparation from the IgG depleted serum. From the control samples, almost no antibody could be isolated indicating near complete depletion of serum IgG.

**Table 1: Representation of the amount of obtained antibody.**

| Sample ID | Average absorbance 280nm | Protein conc. (mg/ml) |
|---|---|---|
| Control | 0.174 | 0.107 |
| Ab1 | 16.542 | 10.211 |

### Quantification of the isolated IgG

The eluted antibody from the serum was concentrated in a 30k Amicon Ultra (0.5ml) column. The final concentrate was quantified for charge variant distribution by cation exchange chromatography.

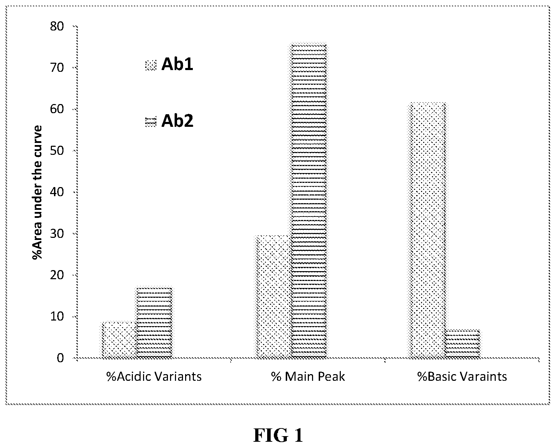

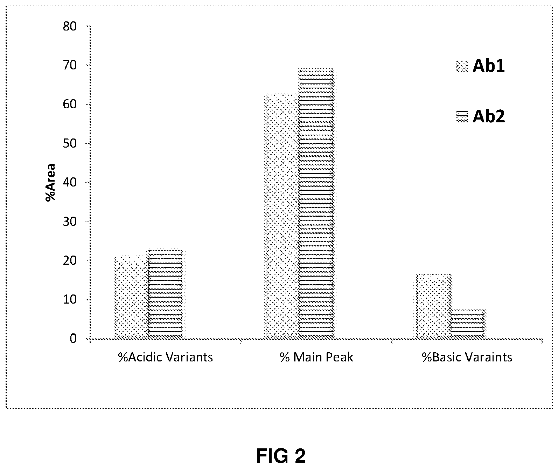

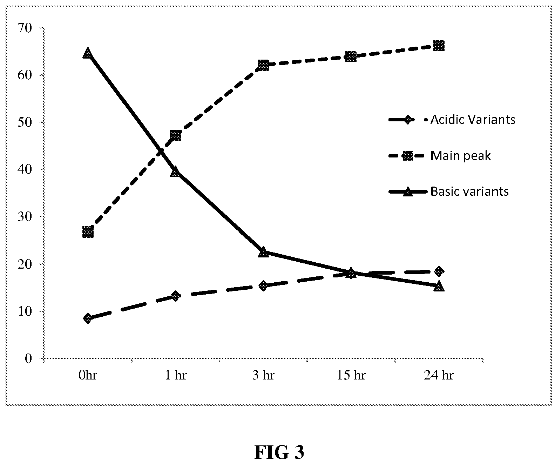

Quantification of the charge variants of Ab1 and Ab2 demonstrated that prior to incubation of the immunoglobulin depleted serum Ab1 contained about 9% acidic variant and about 61% basic variants and the main peak consisted of only about 30% (Figure 1). Interestingly enough, post incubation of the antibody preparations with immunoglobulin depleted serum contained equivalent proportions of acidic, basic or neutral variants of the protein (Figure 2). Finally, it can be observed that, upon incubation of the antibody preparation with the immunoglobulin depleted serum, within three hours about 70% of the basic variants are cleared, and this decline is associated with equivalent increase in the proportion of the main variant (Figure 3).

## Claims

1. An *in* vitro method of determining the fate of immunoglobulin charge variants of a polypeptide preparation, comprising;
a) isolating serum or plasma from blood;
b) depleting immunoglobulin from the said serum or plasma;
c) spiking the immunoglobulin depleted serum or plasma with the polypeptide preparation
d) incubating the spiked serum or plasma of step c for a period of time
e) isolating immunoglobulin and immunoglobulin charge variants thereof from the spiked serum or plasma of step d, and
f) quantifying the immunoglobulin and immunoglobulin charge variants isolated in step e.

2. The method as claimed in claim 1, wherein, the polypeptide preparation is a therapeutic polypeptide preparation

3. The method as claimed in claim 2, wherein, the therapeutic polypeptide preparation is an immunoglobulin preparation.

## Patentansprüche

1. *In*-*vitro*-Verfahren zum Bestimmen des Schicksals von Immunglobulinladungsvarianten eines Polypeptidpräparats, das Folgendes beinhaltet:
a) Isolieren von Serum oder Plasma von Blut;
b) Verarmen von Immunglobulin aus dem genannten Serum oder Plasma;
c) Spicken des immunglobulinverarmten Serums oder Plasmas mit dem Polypeptidpräparat;
d) Inkubieren des gespickten Serums oder Plasmas von Schritt c für eine Zeitperiode;
e) Isolieren von Immunglobulin und Immunglobulinladungsvarianten davon von dem gespickten Serum oder Plasma aus Schritt d, und
f) Quantifizieren des Immunglobulins und von in Schritt e isolierten Immunglobulinladungsvarianten.

2. Verfahren nach Anspruch 1, wobei das Polypeptidpräparat ein therapeutisches Polypeptidpräparat ist.

3. Verfahren nach Anspruch 2, wobei das therapeutische Polypeptidpräparat ein Immunglobulinpräparat ist.

## Revendications

1. Procédé in vitro de détermination du devenir de variants de charge d'immunoglobuline d'une préparation polypeptidique, comprenant :
a) l'isolation du sérum ou plasma du sang ;
b) l'appauvrissement de l'immunoglobuline dudit sérum oh plasma ;
c) le dopage du sérum ou plasma appauvri en immunoglobuline avec la préparation polypeptidique ;
d) l'incubation du sérum ou plasma dopé de l'étape c pendant une période de temps ;
e) l'isolation de l'immunoglobuline et des variantes de charge d'immunoglobuline de celle-ci du sérum ou plasma dopé de l'étape, et
f) la quantification de l'immunoglobuline et des variants de charge d'immunoglobuline isolés à l'étape e.

2. Procédé selon la revendication 1, dans lequel la préparation polypeptidique est une préparation polypeptidique thérapeutique.

3. Procédé selon la revendication 2, dans lequel la préparation polypeptidique est une préparation d'immunoglobuline.
